# EUROPEAN PATENT APPLICATION

(11) **EP 4 537 691 A1**
(43) Date of publication of application: **16.04.2025**
(21) Application number: 24203955.0
(22) Date of filing: 01.10.2024
(51) Int. Cl.: A24F 40/53, A61M 11/04, A61M 15/06

(54) **VAPORIZABLE MATERIAL CONSUMPTION STATE DETECTING METHOD AND VAPORIZER DEVICE**

(30) Priority: 09.10.2023 CN 202311302858
(71) Applicant: Smoore International Holdings Limited, George Town, Grand Cayman, KY1-1111 (KY)
(72) Inventor: YANG, Sheng, Shenzhen Guangdong, 518102 (CN); LUO, Chen, Shenzhen Guangdong, 518102 (CN)
(74) Representative: Westphal, Mussgnug & Partner, Patentanwälte mbB

(57) **Abstract**

According to embodiments of the disclosure, a vaporizable material consumption state detecting method and an vaporizer device are provided. The method includes: monitoring an electrical resistance of a heating element for heating a vaporizable material during a first atomization operation period of the vaporizable material; determining, based on the monitoring, a cumulative amount of a difference between the monitored electrical resistance and a reference electrical resistance over time, wherein the reference electrical resistance indicates the electrical resistance of the heating element that varies over time under a condition that the vaporizable material is sufficient; and detecting, based on the cumulative amount, a consumption state of the vaporizable material during the first atomization operation period. As such, the accuracy of identifying the consumption state of the vaporizable material can be enhanced.

## Description

### TECHNICAL FIELD

Illustrative embodiments of the disclosure generally relate to the field of vaporizer devices, and more particularly to a vaporizable material consumption state detecting method and a vaporizer device.

### BACKGROUND OF THE ART

The population of consumers for atomization equipment, such as electronic vaporizer device, has gradually increased in the market in recent years. With the development of the market and changes in user needs, this trend is expected to continue to grow. In atomization equipment, a heating element is used to heat the vaporizable material for atomization. If the vaporizable material has been depleted and the heating element is still heating, dry burning of the vaporizer device will occur.

### SUMMARY OF THE INVENTION

Embodiments of the disclosure provide a vaporizable material consumption state detecting method and a vaporizer device.

In a first aspect of the disclosure, a vaporizable material consumption state detecting method is provided. The method comprises: monitoring an electrical resistance of a heating element for heating a vaporizable material during a first atomization operation period of the vaporizable material; determining, based on the monitoring, a cumulative amount of a difference between the monitored electrical resistance and a reference electrical resistance over time, wherein the reference electrical resistance indicates the electrical resistance of the heating element that varies over time under a condition that the vaporizable material is sufficient; and detecting, based on the cumulative amount, a consumption state of the vaporizable material during the first atomization operation period.

In some embodiments of the first aspect, the determining of the cumulative amount comprises: determining a characteristic time point based on a change trend of the monitored electrical resistance, wherein the change trend changes at the characteristic time point; and cumulatively determining a difference between the monitored electrical resistance and the reference electrical resistance at sampling time intervals starting from the characteristic time point.

In some embodiments of the first aspect, a stage anterior to the characteristic time point corresponds to a temperature rise stage of the vaporizable material, and a stage posterior to the characteristic time point corresponds to a thermal equilibrium stage of the vaporizable material.

In some embodiments of the first aspect, the detecting of the consumption state of the vaporizable material during the first atomization operation period comprises: determining that the vaporizable material is in an insufficient state during the first atomization operation period in response to the cumulative amount exceeding a first threshold, and the method further comprises at least one of the following: presenting an alarm message that the vaporizable material is insufficient, or making the heating element stop heating the vaporizable material.

In some embodiments of the first aspect, the method further comprises: monitoring the electrical resistance of the heating element during a second atomization operation period anterior to the first atomization operation; determining an electrical resistance difference between the electrical resistance monitored during the second atomization operation period and the reference electrical resistance, and wherein the determining that the vaporizable material is in an insufficient state during the first atomization operation period comprises: determining the vaporizable material is in an insufficient state during the first atomization operation period in response to the electrical resistance difference exceeding a second threshold and the cumulative amount exceeding the first threshold.

In some embodiments of the first aspect, the method further comprises: executing at least one of the following in response to the electrical resistance difference exceeding the second threshold: making the heating element stop heating the vaporizable material, or presenting an early alarm message regarding the consumption state of the vaporizable material.

In some embodiments of the first aspect, the method further comprises: in case that the vaporizable material is detected to be in a sufficient state during the first atomization operation period, substituting the reference electrical resistance with the electrical resistance varying over time and monitored during the first atomization operation period to be used in a third atomization operation period posterior to the first atomization operation.

In a second aspect of the disclosure, an vaporizer device is provided, comprising: a heating element, which is configured for heating a vaporizable material; and a processor, which is configured for: monitoring an electrical resistance of the heating element during a first atomization operation period of the vaporizable material; determining, based on the monitoring, a cumulative amount of a difference between the monitored electrical resistance and a reference electrical resistance over time, wherein the reference electrical resistance indicates the electrical resistance of the heating element that varies over time under a condition that the vaporizable material is sufficient; and detecting, based on the cumulative amount, a consumption state of the vaporizable material during the first atomization operation period.

In a third aspect of the disclosure, an vaporizer device is provided, comprising: an electrical resistance monitoring module, which is configured for monitoring an electrical resistance of a heating element for heating a vaporizable material during a first atomization operation period of the vaporizable material; a cumulative amount determination module, which is configured for determining, based on the monitoring, a cumulative amount of a difference between an electrical resistance monitored thereby and a reference electrical resistance over time, wherein the reference electrical resistance indicates the electrical resistance of the heating element that varies with time under a condition that the vaporizable material is sufficient; and a consumption state detection module, which is configured for detecting, based on the cumulative amount, a consumption state of the vaporizable material during the first atomization operation period.

It will be understood from the following description that according to embodiments of the present disclosure, a vaporizable material consumption state detecting method is provided for an vaporizer device. During a first atomization operation period of a vaporizable material, by monitoring an electrical resistance of a heating element for heating a vaporizable material, an accumulated amount of a difference between the monitored electrical resistance and a reference electrical resistance over time can be determined, so as to detect a consumption state of the vaporizable material during the first atomization operation period. In this way, the accuracy of detecting the consumption state of the vaporizable material can be improved. Other beneficial efficacy will be described below with the development of corresponding embodiments.

It should be understood that the contents described in the detailed description section are not intended to limit the key features or important features of the embodiments of the disclosure, nor are they intended to limit the scope of the disclosure. Other features of the disclosure will become apparent from the description below.

### BRIEF DESCRIPTION OF THE DRAWINGS

The above and other features, advantages and aspects of the various embodiments of the disclosure will become more apparent with reference to the following detailed description in conjunction with the accompanying drawings. In the drawings, the same or similar reference numerals refer to the same or similar elements, wherein:
FIG. 1 shows a schematic view of an illustrative example of a vaporizer device according to the embodiments of the disclosure;
FIG. 2 shows a flow chart of a vaporizable material consumption state detecting method according to some embodiments of the disclosure;
FIG. 3 shows a curve diagram of a monitored electrical resistance and a reference electrical resistance that vary over time according to some embodiments of the disclosure;
FIG. 4 shows a curve diagram of variation of an electrical resistance of a heating element during a normal heating process of the vaporizer device according to some embodiments of the disclosure;
FIG. 5 shows a schematic diagram of determining a characteristic time point according to some embodiments of the disclosure;
FIG. 6 shows a flow chart of a process for detecting a consumption state of a vaporizable material according to some embodiments of the disclosure;
FIG. 7 shows a schematic diagram of a difference between a real-time electrical resistance and a reference electrical resistance according to some embodiments of the disclosure;
FIG. 8 shows a schematic diagram of a cumulative amount and an electrical resistance difference according to some embodiments of the disclosure; and
FIG. 9 shows a block diagram of a vaporizer device that implements various embodiments of the disclosure.

### DETAILED DESCRIPTION OF PREFERRED EMBODIMENTS

Embodiments of the disclosure will be described below, in a more detailed manner, with reference to the attached drawings. Although the drawings illustrate some embodiments of the disclosure, it is appreciated that the disclosure can be implemented in various forms and should not be construed as being limited to the embodiments illustrated herein, and instead, these embodiments are provided for throughout and complete understanding of the disclosure. It is appreciated that the drawings and the embodiments of the disclosure are illustrative only and are not intended to limit the scope of protection that the disclosure pursues.

In the description of the embodiments of the disclosure, the term "comprising" and similar expressions shall be understood as an open inclusion, that is "comprising but not limited to". The term "based on" should be understood to mean "at least partly based on". The terms "one embodiment" or "the embodiment" should be understood to mean "at least one embodiment". The term "some embodiments" should be understood to mean "at least some embodiments". Other explicit and implicit definitions may be included below. The terms "first," "second," and so on may refer to different or the same object. Other explicit and implicit definitions may be included below.

At present, dry burning of an atomizer of an electronic vaporizer device refers to that during use, a heating element inside the atomizer does not have enough vaporizable material to heat, causing the vaporizable material inside the atomizer to begin to burn or dry out. When the electronic vaporizer device detects the consumption status of the vaporizable material, because the heating element (such as a heating filament) is not completely covered by the vaporizable material, resulting in the heating element having no enough vaporizable material to convert into aerosol. In this case, as the electronic vaporizer device cannot accurately detect the consumption state of the vaporizable material, a burnt smell of the vaporizable material may be produced, and even causing damage to the heating element.

The atomizer of traditional electronic vaporizer devices generally uses temperature detection technology to detect the consumption state of the vaporizable material. The following three methods are commonly adopted to detect the consumption state of the vaporizable material in order to prevent the occurrence of dry burning. One is to calculate the temperature of the heating element by monitoring the value of the temperature coefficient of resistance (TCR), in order to then prevent dry burning by controlling the temperature. The second is to identify whether there is dry burning by monitoring the rate of temperature rise. The third is to identify the occurrence of dry burning by monitoring abrupt change in the temperature of the heating filament.

The impedance of a heating element of an electronic vaporizer device is small, and there is a certain deviation in the consistency of heating elements during the production process. For example, the electrical resistance of the heating element is within 1Ω, and the deviation of the consistency is about ±10%. When the atomizer is mounted in the electronic vaporizer device, there is contact impedance for contact of electrodes, and this affects the overall impedance for heating. However, preventing dry burning through temperature controlling requires high consistency in the temperature coefficient of impedance of the heating element, and also, the contact impedance has a great influence on the overall impedance for heating. Therefore, the electronic vaporizer device cannot accurately measure the consumption state of the vaporizable material.

In addition, the specific heat capacity of the vaporizable material itself affects the temperature rise curve and thermal equilibrium temperature. During the course of use of the electronic vaporizer device, the angle of the atomizer and the flow rate of the vaporizable material affect the balance of heating induced by the heating element. However, monitoring the temperature rise rate to identify and prevent the occurrence of dry burning is greatly influenced by the specific heat capacity of the vaporizable material itself, and is only suitable for cold start of the atomizer and is not suitable for continuous vaping. Further, monitoring abrupt change of temperature to identify and prevent the occurrence of dry burning requires multiple times of learning of the thermal equilibrium temperature, and the identification cycle is long. In case that the vaporizable material in the atomizer is insufficient during the course of learning, the learning value will become meaningless. In this way, the consumption state of the vaporizable material cannot be accurately detected. The above describes the problem of inaccurate measurement of the consumption state of the vaporizable material in the electronic vaporizer device. It should be understood that similar problems in the detection of the consumption state of the vaporizable material exist in other types of vaporizer devices.

To this end, embodiments of the disclosure provide a solution for detecting the consumption state of the vaporizable material in the vaporizer device. In the vaporizer device, during a period of a first atomization operation of the vaporizable material, the electrical resistance of the heating element for heating the vaporizable material is monitored. Therefore, a cumulative amount of a difference between the electrical resistance so monitored and a reference electrical resistance over time is determined in order to detect the consumption state of the vaporizable material during the period of the first atomization operation. In this way, the vaporizer device can accurately monitor the consumption state of the vaporizable material to prevent scorch of the vaporizable material resulting from dry burning of the heating element and even to prevent the occurrence of damage of the heating element resulting from dry burning of the heating element. In this way, the reliability of the vaporizer device is enhanced, and the user experience is improved.

Some illustrative embodiments of the disclosure will be described below with reference to the accompanying drawings. It is noted that in some descriptions of the embodiments, some specific numerical values may be involved in order to better help readers understand. These numerical values are illustrative and may vary according to specific application scenarios, and do not limit the scope of the disclosure in any way.

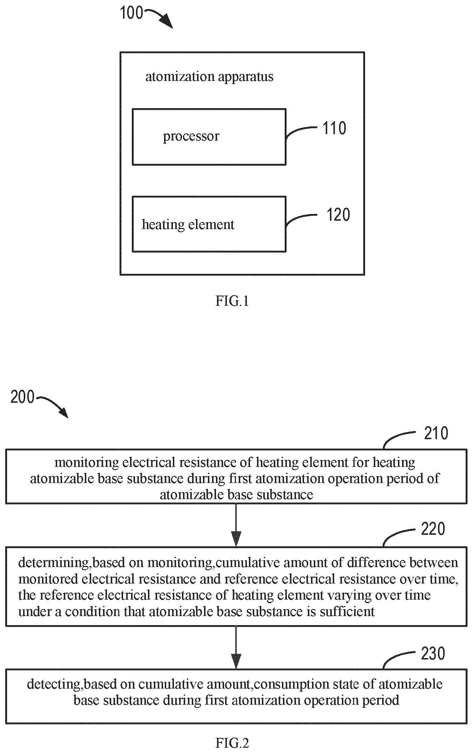

FIG. 1 is a schematic view showing an example of an vaporizer device 100 according to an embodiment of the disclosure. As shown in FIG. 1, the vaporizer device 100, which can be an electronic vaporizer device, comprises a processor 110 and a heating element 120. The processor 110 is coupled to the heating element 120 in order to acquire the electrical resistance of the heating element 120 for detecting a consumption state of a vaporizable material. The heating element 120 is used to heat the vaporizable material during an atomization operation period. For example, the vaporizable material may comprise an oily vaporizable material or a liquid vaporizable material. The heating element 120 may comprise a heating filament, a heating membrane, and a heating circuit, and the embodiment of the disclosure does not impose any limit thereto.

In some embodiments, the vaporizer device 100 further comprises a power supply, an atomizer receiving the vaporizable material and the heating element 120, and a mouth piece. The mouth piece can be integrally formed with another portion (such as a housing) of the vaporizer device 100, or can be separately made and detachably mounted to another portion of the vaporizer device 100. The power supply (such as a battery) functions to supply electrical power to the vaporizer device 100. When a user presses down an activation button or make a vaping, the vaporizer device 100 heats the vaporizable material in the atomizer to convert it into an aerosol, and user may inhale the aerosol through the mouth piece.

The processor 110 can be a piece of electronic equipment of any type and capable of computation. The processor 110 can be for example a microprocessor (MPU), a system-on-chip (SOC), a central processing unit (CPU), and a microcontroller (MCU), and the likes. In the embodiment of the disclosure, the processor 110 executes the vaporizable material consumption state detecting method. The processor 110 may identify the consumption state of the vaporizable material in the heating element 120 to prevent the occurrence of dry burning of the atomizer, in order to prevent scorch of the vaporizable material and damages of the heating element 120 resulting from dry burning of the atomizer.

It should be understood that the structure and function of various parts of the environmental vaporizer device 100 are described for illustrative purposes only and do not imply any limitation on the scope of the disclosure.

Some illustrative embodiments of the disclosure will be described in detail below with reference to the examples illustrated in the accompanying drawings.

FIG. 2 is a flow chart illustrating a vaporizable material consumption state detecting method 200 according to some embodiments of the disclosure. The method 200 is implementable in the processor 110. The method 200 will be described below with reference to FIG. 2.

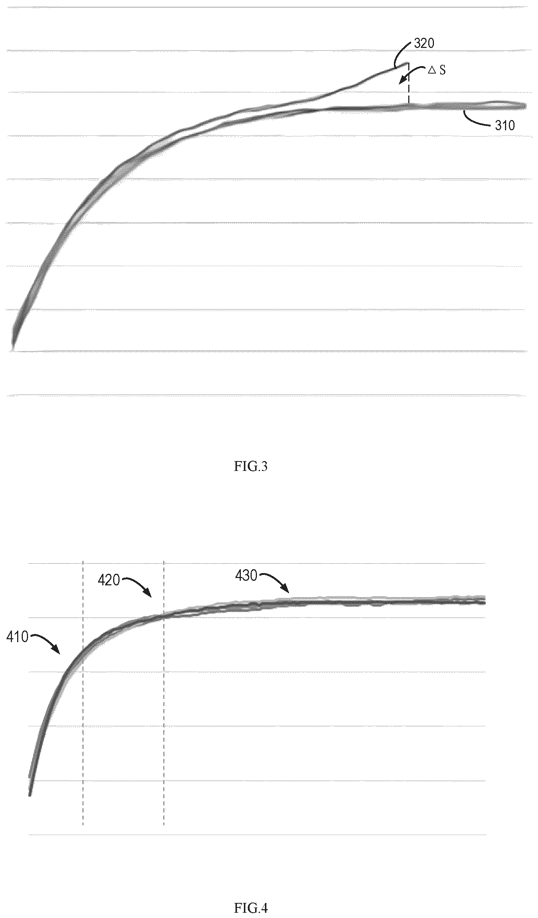

In Block 210, during the first atomization operation period of the vaporizable material, the processor 110 monitors the electrical resistance of the heating element 120. The heating element 120 is used to heat the vaporizable material to form an aerosol that is inhalable by the user as described above. For example, the electrical resistance can be represented by a curve, which will also be referred to as a real-time curve. As an example, FIG. 3 shows a real-time curve of the electrical resistance that is so monitored. As shown in FIG. 3, the real-time curve 320 is a curve of variation of the electrical resistance over time.

In some embodiments, the first atomization operation may be any atomization operation on the vaporizable material during the course of use of the vaporizer device 100. For example, in the case where the vaporizer device 100 is an electronic vaporizer device, the vaporizable material may be an oily vaporizable material in the electronic vaporizer device, and the atomization operation may be a vaping action on the aerosol produced by heating the oily vaporizable material. In this example, the first atomization operation can be understood any puff that the user takes for a vaping action of the electronic vaporizer device.

The electrical resistance of the heating element 120 may be obtained in any suitable manner. For example, the electrical resistance may be detected by a circuit including the heating element 120. Embodiments of the disclosure are not limited in this regard.

In Block 220, the processor 110 determines a cumulative amount of a difference over time between the electrical resistance so monitored and a reference electrical resistance based on the monitoring of Block 210. The reference electrical resistance is indicative of the electrical resistance of the heating element 120 that varies with time under a condition where the vaporizable material is sufficient. For example, the reference electrical resistance may be the electrical resistance of the heating element 120 that varies over time as being monitored during the first-time atomization operation period when the vaporizer device 100 is initially started. As another example, the reference electrical resistance may be the electrical resistance that varies over time as being monitored or collected during the previous period of normal atomization operation as that described below.

FIG. 3 shows a curve of the reference electrical resistance varying over time, which is a reference curve 310. The processor 110 saves the electrical resistance of the heating element 120 that varies with time as being monitored during the first atomization operation period when the vaporizer device 100 is being in the atomization operation as a real-time curve 320. The reference curve 310 is used for comparison with the real-time curve 320. Specifically, the cumulative amount is the integral of the difference between the real-time curve 320 and the reference curve 310 at each time point. In this way, by calculating the integral of the difference between the real-time curve 320 and the reference curve 310, whether the heating element 120 is in a dry burning state can be identified, meaning the vaporizable material is in an insufficient state during the first atomization operation period. In some embodiments, the cumulative amount may be the integral of the difference between the real-time curve 320 and the reference curve 310 calculated from the beginning of the first atomization operation.

In some embodiments, the calculation of the cumulative amount may occur after a characteristic time point. The characteristic time point refers to a time point when the change trend of the electrical resistance changes. Specifically, the processor 110 may determine the characteristic time according to the change trend of the electrical resistance monitored thereby. For example, the characteristic time point may be an inflection point. In some embodiments, the stage anterior to the characteristic time point corresponds to a temperature rise stage 410 of the vaporizable material, and the stage posterior to the characteristic time point corresponds to a thermal equilibrium stage 430 of the vaporizable material. Specifically, referring to FIG. 4, an illustrative example of the characteristic time point is illustrated. The change trend of the electrical resistance (which is also referred to as a resistance variation curve) monitored by the processor 110 is divided into three stages. The three stages are respectively referred, in sequence, to a temperature rise stage 410, an inflection point stage 420, and a thermal equilibrium stage 430. By identifying the inflection point, the change trend of the electrical resistance monitored by the processor 110 is divided into the three stages.

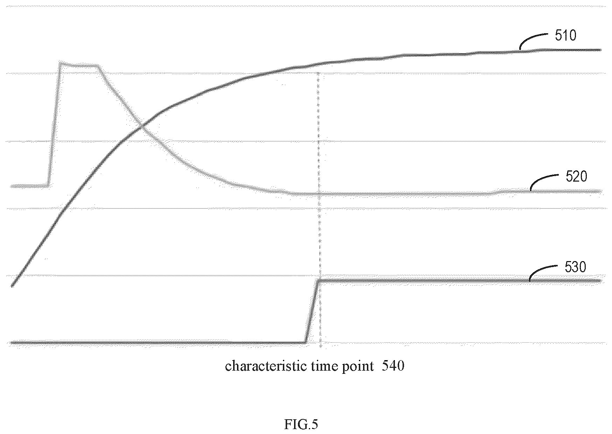

Continuing the above-described process of determining the characteristic time point according to the monitored change trend of the electrical resistance, after heating by the heating element 120 is started, the temperature rises rapidly and reaches equilibrium after reaching a certain temperature. The intermediate process for the heating element 120 proceeding from the temperature rise stage 410 to the equilibrium stage 430 is defined as the inflection point stage 420 of the curve of the change trend of the electrical resistance. For example, referring to FIG. 5, the processor 110 can calculate the slope of five adjacent sampling points of Curve 510 of the change trend of the electrical resistance, and averaging is carried out to obtain a first derivative. The processor 110 can the calculate a second derivative 520 according to the first derivative. The processor 110 constantly tracks the result of the second derivative 520. When the second derivative 520 of three consecutive points approaches 0, it is determined to be the characteristic time point 540, namely the inflection point, referring to Curve 530 shown in FIG. 5. By examining the second derivative 520 of the Curve, the characteristic time point 540 of the Curve can be identified. Understandably, the value of the second derivative 520 is the largest when heating by the heating element 120 is just started, and with heating continuing, the second derivative 520 is gradually reduced to eventually approach 0. At this time, it is considered the characteristic time point 540 of Curve 510 of the change trend of the electrical resistance appears.

In other words, after the atomization operation is started, when the change trend of the electrical resistance of the heating element 120 is in the temperature rise stage 410, the temperature of the heating element 120 rises rapidly, with the initial temperature rise rate being fastest and gradually slowing down, the slope of Curve 510 of the change trend of the electrical resistance being positive, the second derivative 520 being negative. When the change trend of the electrical resistance is at the characteristic time point 540, the temperature change of the heating element 120 becomes slowing down, and the slope of Curve 510 of the change trend of the electrical resistance is positive and the second derivative 520 changes from a negative number to 0 or a positive number and fluctuates around 0. If the change trend of the electrical resistance is in the thermal equilibrium stage 430, the energy provided through heating by the heating element 120 and the dissipation of heat reach a balance, and the temperature changes very slowly, showing a weak trend of rising, and the slope of Curve 510 of the change trend of the electrical resistance and the second derivative 520 both approach 0.

In some embodiments, the atomization operation of the vaporizer device 100 can be divided into a normal process, an intermediate process, and a dry burning process. Specifically, when the vaporizer device 100 is performing the normal process, the vaporizable material is sufficient and conduction of the oil is sufficient. After the atomization operation is started, heat dissipation from the vaporizable material is quick and the characteristic time point appears in around 300ms, and thermal equilibrium can quickly be reached at a normal atomization temperature, no scorch smell. For example, the normal process may be the first atomization operation.

In the intermediate process of the vaporizer device 100, the vaporizable material is in a process from being sufficient to becoming exhausted, and during the first half of the period when the vaporizable material is being consumed, the vaporizable material is supplied sufficiently and the characteristic time point is close to the normal process. However, the heating element 120 enters thermal equilibrium late, or fails to reach thermal equilibrium, and the Curve shows tail up-bending, with slight scorch smell. This is the best time to identify the dry burning of the heating element 120.

In the dry burning process of the vaporizer device 100, there is only a small amount of the vaporizable material on the liquid-conducting cotton of the vaporizer device 100. The temperature of the heating element 120 rises rapidly after starting, and the characteristic time point is later than the normal situation, and the temperature of the characteristic time point and the thermal equilibrium temperature are higher. The thermal equilibrium can also be reached after the characteristic time point appears, but the temperature rising rate is faster than the normal atomization operation, and the equilibrium temperature is high and scorch smell is severe. Even dry burning is detected, the heating core is damaged.

In some embodiments, the processor 110 cumulatively determines the difference between the electrical resistance monitored thereby and the reference electrical resistance at sampling time intervals starting from the characteristic time point. For example, after the characteristic time point of the real-time curve 320 is identified, the difference between the real-time curve 320 and the reference curve 310 is accumulated over time to obtain the cumulative amount (shown as ΔS in the drawings). Specifically, after the characteristic time point appears, the arithmetic difference (shown as ΔR in the drawings) between the real-time curve 320 and the reference curve 310 at each sampling time point is calculated, the sampling time interval being ΔT, and the cumulative amount can be obtained by adding the result of each arithmetic difference, meaning for each sampling time point, ΔS=ΔR×ΔT, and then, ΔS of each sampling time point is accumulated to obtain the cumulative amount.

Continuing to refer to FIG. 2, in Block 230, the processor 110 detects the consumption state of the vaporizable material in the first atomization operation period based on the cumulative amount. In some embodiments, if the cumulative amount exceeds a first threshold, the processor 110 may determine the vaporizable material is in an insufficient state in the first atomization operation period. For example, the insufficient state of the vaporizable material may be a depleted state.

Illustratively, the curve of the change trend of the electrical resistance obtained with atomization performed under a condition where the vaporizable material is sufficient is not a dry burning curve and is used as a reference curve 310 to identify the consumption state of the vaporizable material. The processor 110 stores the values of the reference curve 310 in a numeric set. When the vaporizer device 100 performs an atomization operation again, the characteristic time point of the real-time curve 320 is first detected. After the characteristic time point appears, the cumulative amount is constantly calculated. When the cumulative amount within a period of time exceeds the first threshold (which is a preset fixed threshold (ΔS_MAX)), it is determined that the vaporizable material is in an insufficient state in the first atomization operation period, and that is, there is a potential risk of causing the dry burning situation described above.

In this way, misidentification by the processor 110 caused by translation of the real-time curve 320 (which may also be referred to as a heating curve) resulting from different starting temperatures of the vaporizer device 100 can be avoided. The processor 110 does not need to carry out learning for multiple times, meaning there is no need to monitor the electrical resistance of the heating element 120 multiple times, and inaccurate leaning values resulting from excessively few vaporizable material can be avoided and the algorithm logic can be simplified.

In some embodiments, if it is determined that the vaporizable material is in an insufficient state during the first atomization operation period, the processor 110 may present an alarm message indicating the vaporizable material is insufficient. The processor 110 may also make the heating element 120 stopping heating the vaporizable material. In this way, situations of dry burning of the heating element 120 and even damaging to the heating element 120 can be prevented. For example, the alarm message can be implemented by connecting the processor 110 to alarm devices, such as an audio siren or a flashing light siren. When the processor 110 triggers the condition that the vaporizable material is in an insufficient state during the first atomization operation period, the alarm device emits a sound or flash light signal to attract the attention of the users.

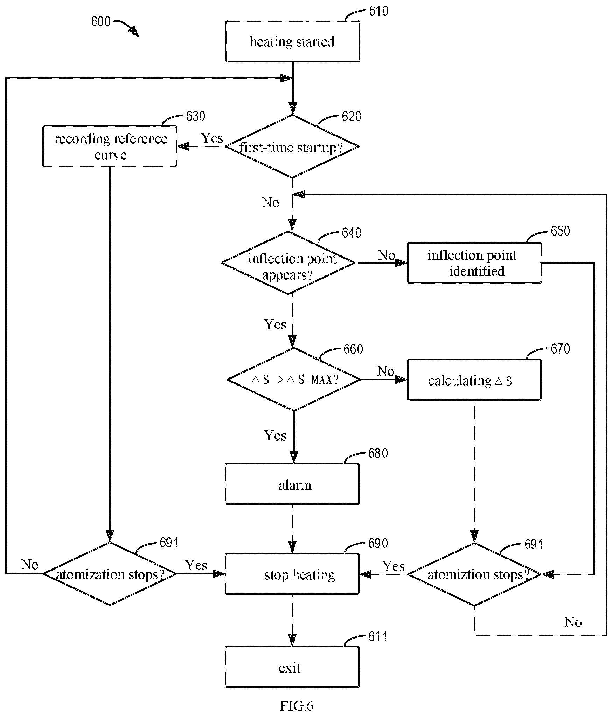

FIG. 6 shows a flowchart of Process 600 for detecting a vaporizable material consumption state according to some embodiments of the disclosure. In some embodiments, Process 600 may be executed in the processor 110 shown in FIG. 1. It should be understood that Process 600 may further comprise additional blocksnot shown and/or omit one (or some) block, and the scope of the disclosure is not limited in this regard.

In Block 610, the heating element 120 is heated and activated. For example, in the electronic vaporizer device described above, when a user performs a vaping action, the processor 110 and the heating element 120 are awakened, and the heating element 120 starts to heat up.

In Block 620, the processor 110 determines whether this is a first-time startup. If it is determined to be the first-time startup, the Process 600 proceeds to Block 630.

In Block 630, the processor 110 records the data of the first-time startup varying over time as the reference curve 310. And then, Process 600 proceeds to Block 691, where the processor 110 determines whether the atomization operation stops. If it is determined that the atomization stops, then Process 600 proceeds to Block 690, in which the heating element 120 stops heating. If it is determined that the atomization does not stop, then Process 600 proceeds to Block 620 again.

If it is determined in Block 620 that this is not a first-time startup, then Process 600 proceeds to Block 640. In Block 640, the processor 110 determines whether an inflection point appears, meaning whether the characteristic time point described above appears. If it is determined that no inflection point appears, then Process 600 proceeds to Block 650, the processor 110 performs identification of inflection point. And then, Process 600 proceeds to Block 691, where the processor 110 determines whether the atomization operation stops. If it is determined that the atomization stops, then Process 600 proceeds to Block 690, in which the heating element 120 stops heating.

If it is determined in Block 640 that an inflection point appears, then Process 600 proceeds to Block 660, where the processor 110 determines whether the cumulative amount ΔS exceeds the first threshold ΔS_MAX. If it is determined that the cumulative amount does not exceed the first threshold, then Process 600 proceeds to Block 670 to calculate the cumulative amount. And then, Process 600 proceeds to Block 691, where the processor 110 determines whether the atomization operation stops. If it is determined that the atomization stops, then Process 600 proceeds to Block 690, in which the heating element 120 stops heating. If it is determined that the atomization does not stop, then Process 600 proceeds to Block 640 again.

If it is determined that the cumulative amount exceeds the first threshold, then Process 600 proceeds to Block 680, where the processor 110 raises an alarm. Afterwards, Process 600 proceeds to Block 690, where the heating element 120 stops heating. Process 600 proceeds to Block 611, where the vaporizer device 100 exits the operation.

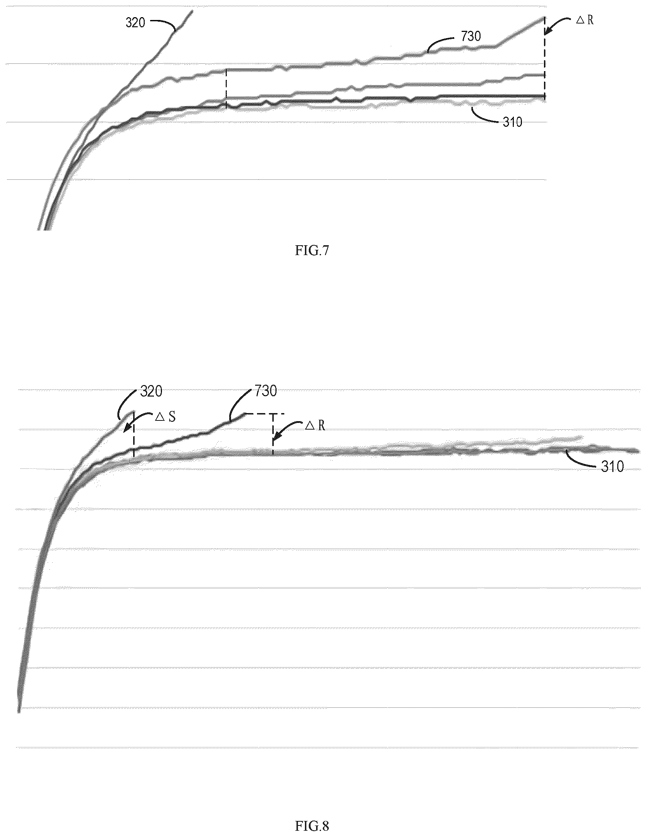

In some embodiments, during a second atomization operation period (which may also be referred to as a previous atomization operation period) before the first atomization operation period, the processor 110 monitors the electrical resistance of the heating element 120. The processor 110 determines an electrical resistance difference between the electrical resistance monitored during the second atomization operation period and the reference electrical resistance. For example, referring to FIG. 7, the processor 110 saves the electrical resistance posterior to the thermal equilibrium stage 430 of the normal curve for the initial startup of the vaporizer device 100 as a reference. The change over time forms the reference curve 310. The difference between the real-time electrical resistance of the second atomization operation period and the reference electrical resistance in the thermal equilibrium stage 430 is ΔR. In this way, the dry burning situation of the vaporizer device 100 is identified by means of the electrical resistance difference. When Curve 730 of the second atomization operation period shows tail up-bending, meaning the electrical resistance difference exceeds the second threshold ΔR_MAX, the heating element 120 stops heating and notifies the user, in order to prevent the high temperature from continuing to damage the vaporizer device 100.

In some embodiments, referring to FIG. 8, the processor 110 determines the vaporizable material is in an insufficient state during the first atomization operation period in response to the electrical resistance difference exceeding the second threshold and the cumulative amount exceeding the first threshold. Based on whether the cumulative amount exceeds the first threshold as described above, in conjunction with identifying whether the electrical resistance difference exceeds the second threshold, whether the vaporizable material is insufficient can be determined. Further, after the atomization operation of the vaporizer device 100 is started, the real-time curve 320 of the first atomization operation does not rapidly enter the thermal equilibrium stage 430 after it rises to the characteristic time point of the second atomization operation, and maintains a trend of rapidly rising, forming a larger included angle with respect to Curve 730 of the second atomization operation, showing obvious dry burning curve characteristics. When the vaporizer device 100 carries out the first atomization operation, if the cumulative amount exceeds the first threshold ΔS_MAX, in combination with the electrical resistance difference exceeding the second threshold ΔR_MAX as described above, the processor 110 can accurately determine that the vaporizer device 100 is dry burning, meaning the vaporizable material is not sufficient, so that it is necessary to stop heating by the heating element 120. By means of the processor 110 identifying the change of the electrical resistance of the heating element 120, the accuracy of the processor 110 identifying the depletion of the vaporizable material can be effectively enhanced. Misidentification caused by solely detecting the threshold of the electrical resistance difference can be avoided to thereby improve the reliability of anti-dry burning detection.

In some embodiments, when the processor 110 responds to the electrical resistance difference exceeding the second threshold, the processor 110 may make the heating element 120 stop heating the vaporizable material. The processor 110 may also present an early alarm message of insufficiency of the vaporizable material. In this way, the heating element 120 is prevented from causing situations of dry burning or even damage to the heating element 120. For example, the early alarm message can be realized by the processor 110 being connected to an early alarm device, such as audible early alarm device or a flash light early alarm device.

In some embodiments, if it is detected that the vaporizable material is in a sufficient state during the first atomization operation period, the electrical resistance that is monitored over time during the first atomization operation period is applied to replace the reference electrical resistance to be used in a third atomization operation period (which can be also referred to a next atomization operation period) after the first atomization operation.

In some embodiments, uneven supply of the vaporizable material or poor liquid conduction of the vaporizable material may cause local high temperature of the nesh core of the heating element 120. If the local high temperature of the heating element 120 is caused by insufficiency of the vaporizable material, then the accumulated amount of the third atomization operation will exceed the first threshold. If the local high temperature of the heating element 120 is caused by poor liquid conduction of the vaporizable material, then the accumulated amount of the third atomization operation will generally not exceed the first threshold, but tail up-bending may possibly occurs at the second half of Curve 730 of the atomization operation. Further, if the processor 110 identifies tail up-bending of the second half of Curve 730, meaning the electrical resistance difference exceeds the second threshold, the heating element 120 stops heating and notifies the user to prevent the heating element 120 from continuing the high temperature to damage the mesh core. If the cumulative amount of the third atomization operation exceeds the first threshold, then the processor 110 determines that the vaporizable material of the vaporizer device 100 is insufficient. In this way, it can be effectively distinguished whether the curve tail up-bending and dry burning is caused by poor liquid conduction of the vaporizable material or dry burning is caused by insufficiency of the vaporizable material, and corresponding protective measures can be taken. In the embodiments of the disclosure, it is possible to identify multiple causes of dry burning without adding additional components.

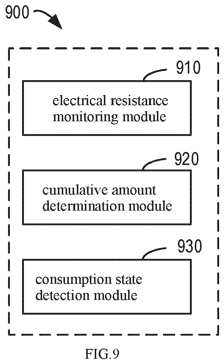

FIG. 9 shows a block diagram of an vaporizer device 900 that implement various embodiments of the disclosure. Each module/component in the vaporizer device 900 can be implemented by hardware, software, firmware, or any combination thereof.

As shown in the drawing, the vaporizer device 900 comprises an electrical resistance monitoring module 910, which is configured for monitoring an electrical resistance of a heating element 120 heating a vaporizable material during a first atomization operation period of the vaporizable material. The vaporizer device 900 further comprises a cumulative amount determination module 920, which is configured for determining, based on the monitoring, a cumulative amount of a difference between an electrical resistance monitored thereby and a reference electrical resistance over time, wherein the reference electrical resistance indicates the electrical resistance of the heating element 120 that varies with time under a condition that the vaporizable material is sufficient. The vaporizer device 900 further comprises a consumption state detection module 930, which is configured for detecting, based on the cumulative amount, a consumption state of the vaporizable material during the first atomization operation period.

In some embodiments, the cumulative amount determination module 920 is configured to determine a characteristic time point according to a change trend of the electrical resistance monitored thereby, wherein the change trend changes at the characteristic time point; and cumulatively determine a difference between the electrical resistance monitored thereby and the reference electrical resistance at sampling time intervals starting from the characteristic time point.

In some embodiments, the stage anterior to the characteristic time point corresponds to a temperature rise stage of the vaporizable material, and the stage posterior to the characteristic time point corresponds to a thermal equilibrium stage of the vaporizable material.

In some embodiments, the consumption state detection module 930 is configured to detect the consumption state of the vaporizable material during the first atomization operation period, and is configured to determine that the vaporizable material is in an insufficient state during the first atomization operation period in response to the cumulative amount exceeding the first threshold. The vaporizer device 900 further comprises at least one of the following: an alarm module, which is configured to present an alarm message that the vaporizable material is insufficient; or a stop module, which is configured to make the heating element stop heating the vaporizable material.

In some embodiments, the electrical resistance monitoring module 910 is configured to monitor the electrical resistance of the heating element during a second atomization operation period that is anterior to the first atomization operation; and determine an electrical resistance difference between the electrical resistance monitored during the second atomization operation period and the reference electrical resistance, and the consumption state detection module 930 is configured to determine that the vaporizable material is in an insufficient state during the first atomization operation period in response to the electrical resistance difference exceeding the second threshold and the cumulative amount exceeding the first threshold.

In some embodiments, the vaporizer device 900 further comprises at least one of the following: a heating stop module, which is configured to make the heating element 120 stop heating the vaporizable material in response to the electrical resistance difference exceeding the second threshold, or an alarm module, which is configured to present an early alarm message regarding the consumption state of the vaporizable material.

In some embodiments, the vaporizer device 900 further comprises a reference electrical resistance substitute module, which is configured to substitute the reference electrical resistance with the electrical resistance that is monitored during the first atomization operation period to vary over time when the vaporizable material is detected to be in a sufficient state during the first atomization operation period, in order to be used in a third atomization operation period posterior to the first atomization operation.

The above provides a description to implementations of the disclosure. The above description is illustrative, not exhaustive, and is not limited to the implementations disclosed herein. For those of ordinary skill in the art, various modifications and variations are easily contemplated without departing from the scope and spirit of the implementations described herein. The terminology used herein is chosen to best explain the principles of each implementation, practical applications or improvements to the technology in the market, or to enable other persons of ordinary skill in the art to understand the various implementations disclosed herein.

## Claims

1. A method for detecting a consumption state of a vaporizable material, comprising:
monitoring an electrical resistance of a heating element for heating the vaporizable material during a first atomization operation period of the vaporizable material;
determining, based on the monitoring, a cumulative amount of a difference between the monitored electrical resistance and a reference electrical resistance over time, wherein the reference electrical resistance indicates the electrical resistance of the heating element that varies over time under a condition that the vaporizable material is sufficient; and
detecting, based on the cumulative amount, the consumption state of the vaporizable material during the first atomization operation period.

2. The method according to claim 1, wherein the determining of the cumulative amount comprises:
determining a characteristic time point based on a change trend of the monitored electrical resistance, wherein the change trend changes at the characteristic time point; and
cumulatively determining a difference between the monitored electrical resistance and the reference electrical resistance at sampling time intervals starting from the characteristic time point.

3. The method according to claim 2, wherein a stage anterior to the characteristic time point corresponds to a temperature rise stage of the vaporizable material, and a stage posterior to the characteristic time point corresponds to a thermal equilibrium stage of the vaporizable material.

4. The method according to claim 1, wherein the detecting of the consumption state of the vaporizable material during the first atomization operation period comprises:
determining that the vaporizable material is in an insufficient state during the first atomization operation period in response to the cumulative amount exceeding a first threshold, and the method further comprises at least one of the following:
presenting an alarm message that the vaporizable material is insufficient, or
making the heating element stop heating the vaporizable material.

5. The method according to claim 4, further comprising:
monitoring the electrical resistance of the heating element during a second atomization operation period anterior to the first atomization operation;
determining an electrical resistance difference between the electrical resistance monitored during the second atomization operation period and the reference electrical resistance, and
wherein the determining that the vaporizable material is in an insufficient state during the first atomization operation period comprises:
determining the vaporizable material is in an insufficient state during the first atomization operation period in response to the electrical resistance difference exceeding a second threshold and the cumulative amount exceeding the first threshold.

6. The method according to claim 5, further comprising:
executing at least one of the following in response to the electrical resistance difference exceeding the second threshold:
making the heating element stop heating the vaporizable material, or presenting an early alarm message regarding the consumption state of the vaporizable material.

7. The method according to claim 1, further comprising:
in case that the vaporizable material is detected to be in a sufficient state during the first atomization operation period, substituting the reference electrical resistance with the electrical resistance varying over time and monitored during the first atomization operation period to be used in a third atomization operation period posterior to the first atomization operation.

8. A vaporizer device, comprising:
a heating element configured for heating a vaporizable material; and
a processor which is configured for:
monitoring an electrical resistance of the heating element during a first atomization operation period of the vaporizable material;
determining, based on the monitoring, a cumulative amount of a difference between the monitored electrical resistance and a reference electrical resistance over time, wherein the reference electrical resistance indicates the electrical resistance of the heating element that varies over time under a condition that the vaporizable material is sufficient; and
detecting, based on the cumulative amount, a consumption state of the vaporizable material during the first atomization operation period.

9. A vaporizer device, comprising:
an electrical resistance monitoring module which is configured for monitoring an electrical resistance of a heating element for heating a vaporizable material during a first atomization operation period of the vaporizable material;
a cumulative amount determination module, which is configured for determining, based on the monitoring, a cumulative amount of a difference between an electrical resistance monitored thereby and a reference electrical resistance over time, wherein the reference electrical resistance indicates the electrical resistance of the heating element that varies with time under a condition that the vaporizable material is sufficient; and
a consumption state detection module which is configured for detecting, based on the cumulative amount, a consumption state of the vaporizable material during the first atomization operation period.

10. The vaporizer device according to claim 9, wherein the cumulative amount determination module is configured to determine a characteristic time point according to a change trend of the electrical resistance monitored thereby, wherein the change trend changes at the characteristic time point; and cumulatively determine a difference between the electrical resistance monitored thereby and the reference electrical resistance at sampling time intervals starting from the characteristic time point.

11. The vaporizer device according to claim 9, wherein the consumption state detection module is configured to determine that the vaporizable material is in an insufficient state during the first atomization operation period in response to the cumulative amount exceeding the first threshold.

12. The vaporizer device according to claim 11, further comprising at least one of the following features:
an alarm module, which is configured to present an alarm message that the vaporizable material is insufficient;
a stop module, which is configured to make the heating element stop heating the vaporizable material.

13. The vaporizer device according to claim 9, wherein the electrical resistance monitoring module is configured to monitor the electrical resistance of the heating element during a second atomization operation period that is anterior to the first atomization operation; and determine an electrical resistance difference between the electrical resistance monitored during the second atomization operation period and the reference electrical resistance, and the consumption state detection module is configured to determine that the vaporizable material is in an insufficient state during the first atomization operation period in response to the electrical resistance difference exceeding the second threshold and the cumulative amount exceeding the first threshold.

14. The vaporizer device according to claim 13, further comprising at least one of the following features:
a heating stop module, which is configured to make the heating element stop heating the vaporizable material in response to the electrical resistance difference exceeding the second threshold;
an alarm module, which is configured to present an early alarm message regarding the consumption state of the vaporizable material.

15. The vaporizer device according to claim 9, further comprising:
a reference electrical resistance substitute module, which is configured to substitute the reference electrical resistance with the electrical resistance that is monitored during the first atomization operation period to vary over time when the vaporizable material is detected to be in a sufficient state during the first atomization operation period, in order to be used in a third atomization operation period posterior to the first atomization operation.
